# EUROPEAN PATENT APPLICATION

(11) **EP 2 040 064 A1**
(43) Date of publication of application: **25.03.2009**
(21) Application number: 06745936.2
(22) Date of filing: 01.05.2006
(51) Int. Cl.: G01N 27/06, G01N 31/00

(54) **DEVICE FOR MEASURING TOTAL ORGANIC CARBON**

(71) Applicant: Shimadzu Corporation, Kyoto-shi 604-8511 (JP)
(72) Inventor: AKECHI, Masakazu, Kyoto-shi, Kyoto 604-8511 (JP); NAKAMORI, Akioki, Kyoto-shi, Kyoto 604-8511 (JP); MORITA, Yozo, Beijing 100004 (CN)
(74) Representative: Stöckeler, Ferdinand
(86) International application number: PCT/JP2006/309085
(87) International publication number: WO 2007/129383

(57) **Abstract**

A device for measuring total organic carbon includes an organic substance oxidative decomposition section (4) for converting organic carbon contained in sample water into carbon dioxide, a carbon dioxide extraction section (6) for extracting carbon dioxide generated in the organic substance oxidative decomposition section (4) into measurement water, and a detection section for measuring the conductivity of the measurement water discharged from the carbon dioxide extraction section (6) to determine the amount of the extracted carbon dioxide. A syringe pump (12) is provided downstream of the detection section (11) in a measurement flow path (16) through which measurement water flows to pass through the carbon dioxide extraction section 6 and the detection section (11). The flow rate of measurement water during measurement of conductivity in the detection section (12) is controlled by suction into the syringe pump.

## Description

### TECHNICAL FIELD

The present invention relates to a device for measuring total organic carbon for measuring the amount of total organic carbon (TOC) contained in sample water containing few impurities by extracting TOC into measurement water and measuring the conductivity of the measurement water. For example, the present invention relates to a µTAS (Micro Total Analysis System) for analyzing a very small amount of sample by using a very small amount of measurement water.

### BACKGROUND ART

Examples of a liquid sample containing few impurities, called pure water or ultrapure water, include water for pharmaceutical production, process water for semiconductor manufacturing, cooling water, boiler water, and tap water.

As a device for measuring total organic carbon for measuring the total organic carbon content of sample water, there is a measuring device having an organic substance oxidative decomposition section for converting organic carbon into carbon dioxide, a carbon dioxide extraction section for extracting carbon dioxide generated in the organic substance oxidative decomposition section into measurement water through a gas permeable membrane, and a detection section for measuring the conductivity of the measurement water in order to determine the amount of carbon dioxide extracted by the carbon dioxide extraction section.

In the carbon dioxide extraction section of such a device for measuring total organic carbon, measurement water is separated by the gas permeable membrane from sample water having been subjected to oxidation treatment in the organic substance oxidative decomposition section to transfer carbon dioxide from the sample water to the measurement water through the gas permeable membrane (see Patent Document 1).
Patent Document 1: U.S. Patent No. 5132094

### DISCLOSURE OF THE INVENTION

### PROBLEMS TO BE RESOLVED BY THE INVENTION

When the flow rate of measurement water fluctuates, the conductivity of the measurement water measured by the detection section varies so that detection accuracy is lowered.
As a measure to stabilize the flow rate of measurement water flowing through a measurement flow path, providing a flow rate control valve in the measurement flow path is a possibility. However, the operation of controlling the flow rate of measurement water is complicated. Further, when the flow rate of measurement water flowing through a measurement flow path is changed due to, for example, a change in the flow rate of a pump for feeding measurement water with time, it is necessary to adjust the flow rate control valve on each occasion to make the flow rate of measurement water constant If the flow rate control valve is not adjusted, reproducibility is lowered.
Further, if the pump for feeding measurement water has a pulsating flow, the flow rate of measurement water fluctuates. Also in this case, measured values vary greatly.

It is therefore an object of the present invention to provide a device for measuring total organic carbon capable of further stabilizing the flow rate of measurement water flowing through a measurement flow path to accurately measure the conductivity of the measurement water.

### MEANS OF SOLVING THE PROBLEMS

The present invention is directed to a device for measuring total organic carbon including: an organic substance oxidative decomposition section for converting organic carbon contained in sample water into carbon dioxide; a carbon dioxide extraction section for extracting carbon dioxide generated in the organic substance oxidative decomposition section into measurement water through a gas permeable membrane; and a detection section for measuring the conductivity of the measurement water discharged from the carbon dioxide extraction section to determine the amount of the extracted carbon dioxide. In the present invention, a syringe pump is provided downstream of the detection section in a measurement flow path through which the measurement water flows to pass through the carbon dioxide extraction section and the detection section, and the flow rate of the measurement water during measurement of conductivity in the detection section is controlled by suction into the syringe pump.
Examples of the measurement water to be used include deionized water obtained by ion exchange, distilled water, and pure water such as Mili-Q water.

In order to efficiently control the flow rate of measurement water by the syringe pump, it is preferred that the measurement flow path through which measurement water flows to pass through the detection section branches into the syringe pump and a discharge flow path, and on the discharge flow path side located downstream of a branch point between the syringe pump and the discharge flow path in the measurement flow path, an open/close valve controlled to be closed during suction into the syringe pump and to be opened during discharge from the syringe pump is provided.

Alternatively, efficient control of the flow rate of measurement water by the syringe pump may be achieved by branching the measurement flow path through which measurement water flows to pass through the detection section into the syringe pump and a discharge flow path and by providing at a branch point between the syringe pump and the discharge flow path, a switching valve which connects the syringe pump to the detection section during suction into the syringe pump and which connects the syringe pump to the discharge flow path during discharge from the syringe pump.

In a case where measurement water is deionized by an ion exchange resin column, it is often the case that the flow rate of measurement water to be supplied into the ion exchange resin column needs to be larger than that of measurement water to be supplied into the measurement flow path. Therefore, in a case where an ion exchange resin column is provided on the upstream side in a liquid delivery flow path for supplying measurement water into the carbon dioxide extraction section, it is preferred that the liquid delivery flow path branches into the measurement flow path and a bypass flow path at a position located downstream of the ion exchange resin column so that the flow rate of measurement water to be supplied into the measurement flow path through the liquid delivery flow path can be controlled by the split function of the bypass flow path.

Further, in a case where the ion exchange resin column is used, the discharge side of the measurement flow path and the discharge side of the bypass flow path may be connected to the supply side of the ion exchange resin column in order to circulate water used as measurement water.

In order to reduce the size of the carbon dioxide extraction section to be used in the device for measuring total organic carbon according to the present invention, the carbon dioxide extraction section is preferably formed by stacking a gas permeable portion through which carbon dioxide contained in sample water passes, a first substrate fixed to the gas permeable portion so as to be opposed thereto and having a flow path through which sample water flows, and a second substrate fixed to the gas permeable portion so as to be opposed thereto and having a flow path through which measurement water flows.

In order to further reduce the size of the device for measuring total organic carbon, it is preferred that the detection section is formed by providing an electrode in a flow path formed between the second substrate and a third substrate fixed to the second substrate so as to be opposed thereto, and that the organic substance oxidative decomposition section is constituted from a flow path formed between the first substrate and a fourth substrate fixed to the first substrate so as to be opposed thereto and an irradiation portion for irradiating the flow path with UV light

The carbon dioxide extraction section can use, as the gas permeable portion, any one selected from the group consisting of a membrane filter, a porous fluorine membrane, a hollow fiber membrane, and a membrane having a plurality of fine flow paths.

### EFFECTS OF THE INVENTION

A flow rate control valve may be provided in a flow path for measurement water to control the flow rate of measurement water flowing through the carbon dioxide extraction section and the detection section, but this involves a complicated operation to control the flow rate of measurement water. However, according to the present invention, the flow rate of measurement water flowing through the detection section is controlled by suction into the syringe pump instead of using a flow rate control valve. Therefore, unlike the case of using a flow rate control valve, it is not necessary to carry out a complicated operation to control the flow rate of measurement water.
Further, even when the flow rate of measurement water discharged from a pump for feeding measurement water is changed due to a change of the pump with time caused by, for example, aging, the flow rate of measurement water flowing through the detection section is controlled by the flow rate of measurement water sucked into the syringe pump, and is therefore unaffected by fluctuations in the flow rate of the pump for feeding measurement water, thereby improving the reproducibility of measurement

Further, by stacking the carbon dioxide extraction section and the organic substance oxidative decomposition section and/or the detection section, it is possible to produce a device for measuring total organic carbon having a reduced size.

### DETAILED DESCRIPTION OF THE INVENTION

Hereinbelow, some embodiments of the present invention will be described.

### (First Embodiment)

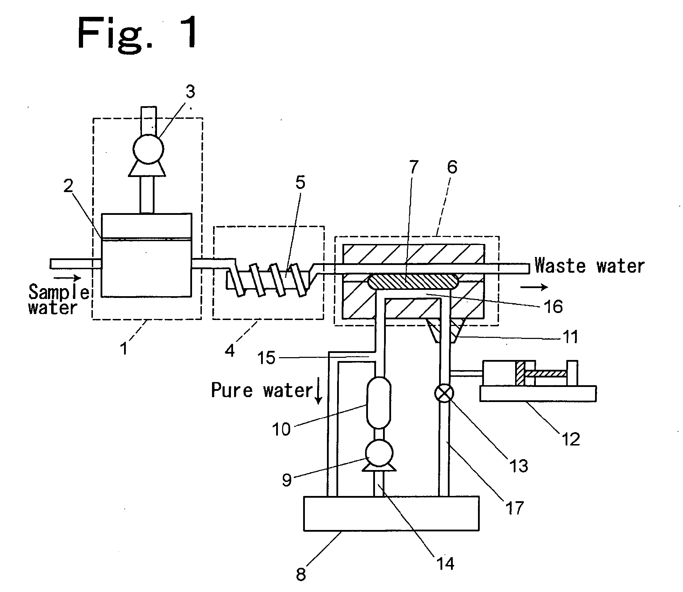

Fig. 1 is a schematic view of a device for measuring total organic carbon.
The device for measuring total organic carbon includes an IC (inorganic carbon) removal section 1 for removing carbon dioxide originally dissolved in sample water, an organic substance oxidative decomposition section 4 for converting organic carbon contained in the sample water into carbon dioxide, a carbon dioxide extraction section 6 for extracting carbon dioxide generated in the organic substance oxidative decomposition section 4 into measurement water, and a detection section 11 for measuring the conductivity of the measurement water discharged from the carbon dioxide extraction section 6 to determine the amount of carbon dioxide extracted by the carbon dioxide extraction section 6.

The IC removal section 1 removes carbon dioxide from sample water through the use of a hydrophobic porous membrane 2 by reducing pressure using a vacuum pump 3. The organic substance oxidative decomposition section 4 has a structure in which a flow path is wound around a UV (ultraviolet ray) lamp 5, and therefore sample water is oxidized by UV energy from the UV lamp 5 while flowing through the flow path. It is preferred that the inner surface of the flow path of the organic substance oxidative decomposition section 4 is coated with a catalyst (e.g., titanium oxide).

The carbon dioxide extraction section 6 has a structure, in which a gas permeable portion 7 is interposed between a flow path for sample water and a measurement flow path 16 for measurement water, to extract carbon dioxide contained in sample water into measurement water. Sample water discharged from the organic substance oxidative decomposition section 4 flows through the flow path for sample water, and measurement water flows through the measurement flow path 16. Measurement water discharged from the carbon dioxide extraction section flows into the detection section 11.

Examples of the gas permeable portion 7 to be used in the carbon dioxide extraction section 6 include a fluorine resin porous membrane having a plurality of pores (e.g., POREFLON® manufactured by Sumitomo Electric Industries, Ltd.), a membrane filter having a plurality of pores (e.g., a polycarbonate membrane filter having a thickness of 10 µm, a pore size of 0.2 µm, and a porosity of 5 to 20 % (ISOPORE MEMBRANE FILTER manufactured by MILLIPORE)), a porous membrane made of PTFE (polytetrafluoroethylene), and a membrane having a plurality of fine flow paths formed in a direction orthogonal to the flow path.

In the measurement flow path 16 through which measurement water flows to pass through the carbon dioxide extraction section 6 and the detection section 11, a flow path located downstream of the detection section 11 branches into a syringe pump 12 and a discharge flow path 17. On the discharge flow path 17 side located downstream of a branch point between the syringe pump 12 and the discharge flow path 17 in the measurement flow path 16, an open/close valve 13 controlled to be closed during suction into the syringe pump 12 and to be opened during discharge from the syringe pump is provided. The measurement flow path 16 located downstream of the open/close valve 13 is connected to a pure water tank 8. The pure water tank 8 is connected to a liquid delivery flow path 14 for delivering measurement water to the carbon dioxide extraction section 6 through an electromagnetic pump 9 and an ion exchange resin column 10.

The syringe pump 12 sucks and discharges a liquid by reciprocating a plunger in a syringe. The amount of a liquid discharged from the syringe pump 12 by one reciprocation of the plunger is determined by the capacity of a space created in the syringe by moving the plunger during suction. Therefore, the syringe pump 12 preferably has a plunger tip which moves together with the plunger so that the syringe can keep close contact with the plunger.

The liquid delivery flow path 14 for delivering measurement water to the carbon dioxide extraction section 6 branches into the measurement flow path 16 and a bypass flow path 15 at a position located downstream of the ion exchange resin column 10. The flow rate of measurement water to be supplied into the measurement flow path 16 is controlled to be very small by the split function of the bypass flow path 15.

Hereinbelow, the operation of the device for measuring total organic carbon according to this embodiment will be described.
An acid is added to sample water containing organic substance, and the sample water is sent to the IC removal section 1. It is difficult to remove carbon dioxide from water because carbon dioxide is dissociated in water. However, addition of an acid prevents dissociation, and therefore it becomes easy to remove carbon dioxide from sample water in the IC removal section 1. In the IC removal section 1, carbon dioxide derived from inorganic substance is removed from the sample water through the hydrophobic porous membrane 2 by reducing pressure using the vacuum pump 3.

Then, the sample water is sent to the organic substance oxidative decomposition section 4 to decompose organic substance through oxidation by UV energy, addition of an oxidizing agent, or a catalyst. As a result, organic substance contained in the sample water is converted into carbon dioxide. The sample water containing dissolved carbon dioxide is sent to the carbon dioxide extraction section 6 to transfer carbon dioxide contained in the sample water into measurement water through the gas permeable portion 7.

Measurement water stored in the pure water tank 8 is pumped up by the electromagnetic pump 9 and flows through the ion exchange resin column 10. Then, part of the measurement water is sent to the measurement flow path 16 connected to the carbon dioxide extraction section 6, and the rest is returned to the pure water tank 8 through the bypass flow path 15. The measurement water containing carbon dioxide extracted by the carbon dioxide extraction section 6 is sent to the detection section 11 to measure the conductivity of the measurement water to determine the carbon dioxide content of the measurement water. As a result, the total organic carbon content of the sample water is determined.

The measurement water discharged from the detection section 11 is sucked into the syringe pump 12 at a constant rate in a state where the open/close valve 13 is closed, and therefore the flow rate of the measurement water flowing through the carbon dioxide extraction section 6 and the detection section 11 is controlled to be constant by the syringe pump 12. After the completion of measurement, the electromagnetic pump 9 is stopped, and then the open/close valve 13 is opened to return the measurement water in the syringe pump 12 to the pure water tank 8 to prepare for the next measurement

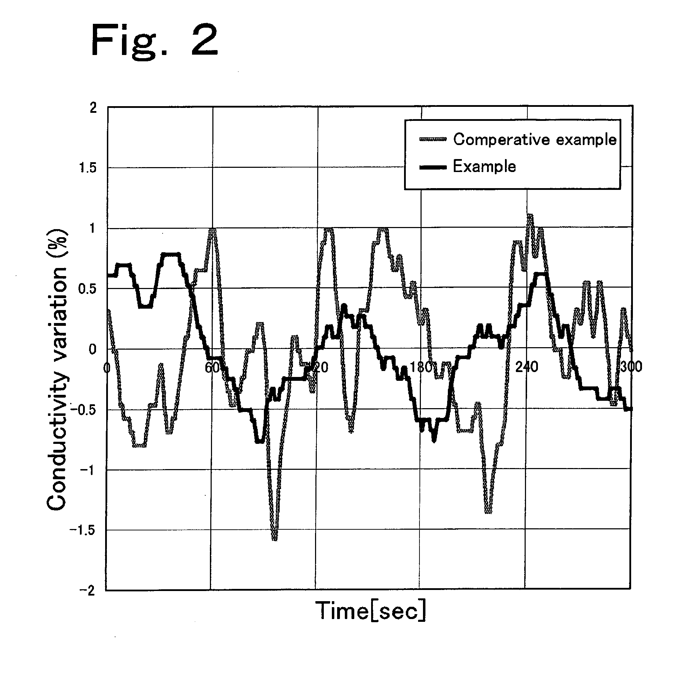

Fig. 2 is a graph showing variations in measured conductivity. In the case of a graph represented as "Example", measurement was carried out by the method described above. More specifically, pure water (water left standing overnight to make the concentration of carbon dioxide the same as that of air) was sent as sample water to the carbon dioxide extraction section 6 at a flow rate of 0.1 mL/min while measurement water was sent to the carbon dioxide extraction section 6 at a flow rate of 0.1 mL/min achieved by splitting the measurement water flowing at a flow rate of 1 mL/min by utilizing the split function of the bypass flow path 15.

The graph shows a change of conductivity with time when the measurement water was sucked into the syringe pump 12 (inner diameter. 7.28 mm, internal length: 60 mm, internal capacity: 2.5 mL) at 0.1 mL/min for 300 seconds. The size of the syringe pump is not limited to one mentioned above, but the syringe pump is preferably thin because an error resulting from the surface of the syringe in close contact with the surface of the plunger becomes small.

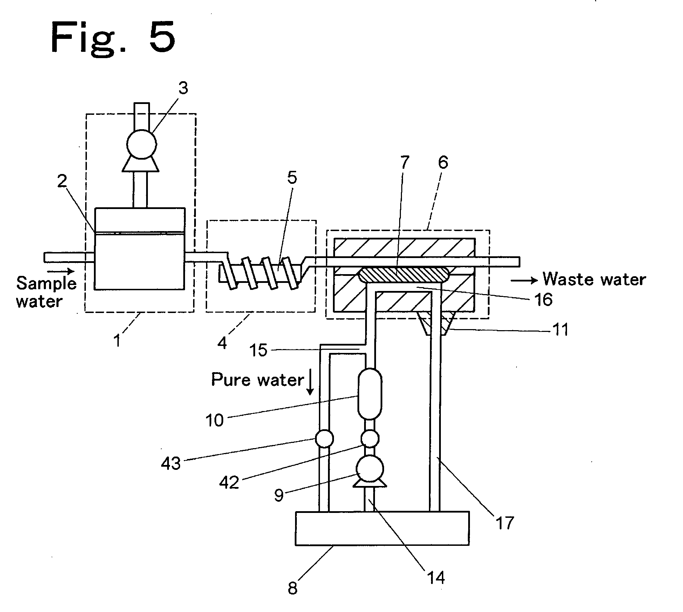

In the case of a graph represented as "Comparative Example", measurement was carried out under the same conditions as in the Example except that the device for measuring total organic carbon according to the present invention was changed to a device shown in Fig. 5. In the device shown in Fig. 5, a needle valve 42 is provided to adjust the flow rate of a commercially available electromagnetic pump 9 to 1 mL/min, and a needle valve 43 is provided in a bypass 15 to adjust the flow rate of measurement water flowing through a conductivity meter 11 to 0.1 mL/min.

In a case where the flow rate of measurement water was controlled by the syringe pump 12 according to the present invention (i.e., in the case of Example), variations in conductivity were within the range of -0.25 to +0.75 % (± 1.0 %). On the other hand, in a case where the flow rate of measurement water was not controlled by the syringe pump 12 (i.e., in the case of Comparative Example), variations in conductivity were within the range of -1.6 to +1.1 %. That is, the measurement result was better in the case of Example than in the case of Comparative Example.

### (Second Embodiment)

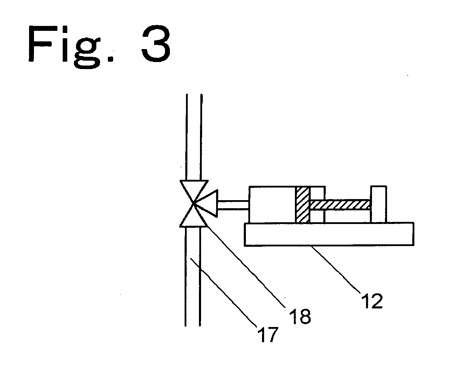

According to the first embodiment, the open/close valve 13 is provided in the discharge flow path 17 located downstream of the detection section 11 in the measurement flow path 16 through which measurement water flows to pass through the detection section 11. On the other hand, according to the second embodiment of the present invention, as shown in Fig. 3, a flow path switching valve 18 is provided instead of the open/close valve 13 at a branch point between the syringe pump 12 and the discharge flow path 17. In this case, switching operation of the flow path switching valve 18 is controlled so that the syringe pump 12 is connected to the detection section 11 during suction into the syringe pump 12, and so that the syringe pump 12 is connected to the discharge flow path 17 during discharge from the syringe pump 12.

### (Third Embodiment)

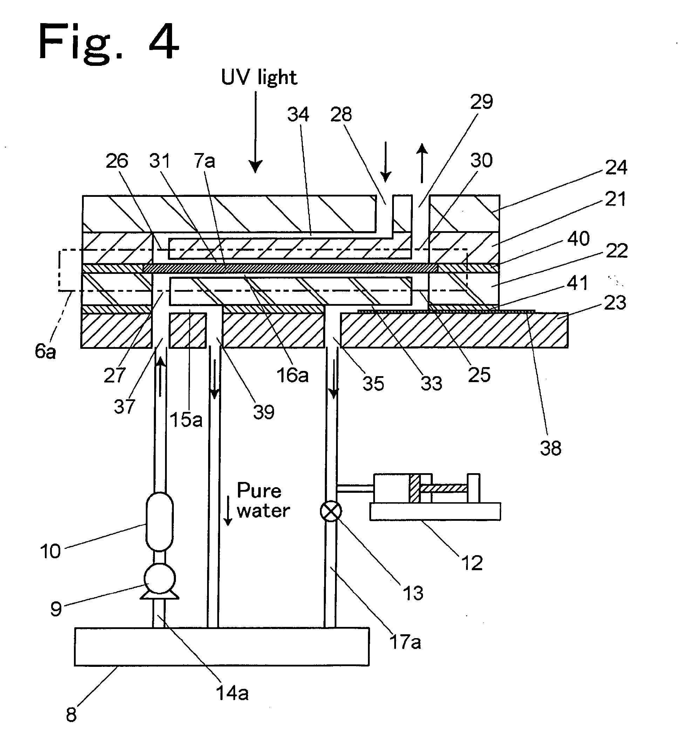

Fig. 4 is a sectional view of a specific embodiment of the device for measuring total organic carbon according to the present invention, in which a carbon dioxide extraction section is formed by stacking substrates to integrate them into one unit
In this total organic carbon measuring device, a carbon dioxide extraction section 6a is sandwiched between an organic substance oxidative decomposition section located on the upper side of the carbon dioxide extraction section 6a and a conductivity measuring section located on the lower side of the carbon dioxide extraction portion 6a so as to be integrated with them.
The carbon dioxide extraction section 6a is formed by stacking a gas permeable portion 7a, a first substrate 21 for forming a sample flow path 31 with the gas permeable section 7a, and a second substrate 22 for forming a measurement flow path 16a on the opposite side of the gas permeable portion 7a from the sample flow path 31 in such a manner that the gas permeable portion 7a is fixed to the first substrate 21 and the second substrate 22.

The sample flow path 31 can be provided by forming a fine groove on the lower surface of the substrate 21 (width: 0.01 to 10 mm, depth: 0.01 to 0.5 mm, length: several millimeters to several hundred millimeters). For example, the fine groove has a width of 1 mm, a depth of 0.2 mm, and a length of 200 mm. The flow path can be formed on a glass substrate by a wet etching technique, a dry etching technique, or a fine processing technique using sandblasting.

The measurement flow path 16a can be provided by forming a fine groove on the upper surface of the substrate 22 (width: 0.01 to 10 mm, depth: 0.01 to 0.5 mm, length: several millimeters to several hundred millimeters). For example, the fine groove has a width of 1 mm, a depth of 0.2 mm, and a length of 200 mm. The measurement flow path 16a can be provided using the same processing technique as used for the sample flow path 31.
As the gas permeable portion 7a, a hydrophobic porous membrane is preferably used. For example, a porous fluorine resin membrane (e.g., POREFLON® manufactured by Sumitomo Electric Industries, Ltd.) or the like can be used.

The conductivity measuring section includes a third substrate 23 fixed to the second substrate 22 so as to be opposed thereto to form a measuring cell, connected to the measurement flow path 16a, with the second substrate 22, and a conductivity measuring electrode 38 provided in the measuring cell. One end of a flow path 33 in the measuring cell is connected to the measuring flow path 16a through a through hole 25, and the other end of the flow path 33 is connected to a discharge port 35 provided as a through hole in the substrate 23.

In the substrate 23, a measurement water supply flow path 37 as a through hole is provided. The supply flow path 37 is connected to the measurement flow path 16a through a through hole 27 provided in the substrate 22, and is also connected to a discharge port 39 provided as a through hole through a bypass flow path 15a formed by the second substrate 22 and the third substrate 23.

The supply flow path 37 for supplying deionized water as measurement water is provided in the substrate 23 at a position corresponding to the through hole 27. Further, the bypass flow path 15a and discharge port 39 for splitting measurement water to discharge a surplus of the measurement water are provided adjacent to the measurement water supply flow path 37 so that the flow rate of measurement water to be introduced into the flow path 33 on the measuring electrode 38 can be controlled. Therefore, it is possible to supply measurement water into an ion exchange resin column 10 while the flow rate of the measurement water is kept optimum so that the ion exchange resin column 10 can be optimally operated.

The flow paths 36 and 33 to be formed between the substrate 22 and the substrate 23 can be realized by creating a space between the substrate 22 and the substrate 23 with the use of PDMS (polydimethylsiloxane: e.g., Silgard 184® manufactured by Coming), an adhesive organic film, or a thin film sheet 41 having an adhesive applied thereto. The depth of each of the flow paths is preferably in the range of 10 to 1000 µm. In this embodiment, these flow paths are formed to have a depth of about 100 µm. The through holes, supply port, and discharge ports to be provided in the substrates 22 and 23 can be formed by, for example, sandblasting.

As shown in Fig. 1, a flow path connected to the discharge port 35 branches into a syringe pump 12 and a discharge flow path 17. In the measurement flow path 16, an open/close valve 13 is provided downstream of a branch point between the syringe pump 12 and the discharge flow path 17. The open/close valve 13 is controlled to be closed during suction into the syringe pump 12 and to be opened during discharge from the syringe pump 12. The measurement flow path 16 located downstream of the open/close valve 13 is connected to a pure water tank 8. The pure water tank 8 is connected to the supply flow path 37 through an electromagnetic pump 9 and the ion exchange resin column 10.

The measuring electrode 38 is formed from a Pt/Ti film (which is obtained by forming a Pt film on a Ti film to improve adhesion) and has, for example, a comb teeth-shaped electrode pattern. Such an electrode pattern can be formed by forming a Pt/Ti film on the silica glass substrate 23 and subjecting the Pt/Ti film to patterning using photolithography and etching in combination. To the surface of the substrate 23 including part of the upper surface of the measuring electrode 38, the adhesive thin film sheet 41, such as PDMS, from which portions corresponding to the flow paths 36 and 33 have been removed, is attached, and then the substrate 22 having the through holes 27 and 25 and the measuring flow path 16a formed as a groove is bonded to the substrate 23 so as to cover the substrate 23.

The organic substance oxidative decomposition section includes a fourth substrate 24 fixed to the first substrate 21 so as to be opposed thereto. Between the first substrate 21 and the fourth substrate 24, an oxidation flow path 34 is formed. As the fourth substrate 24, a substrate made of a material transparent to UV light, such as a silica glass substrate, is used so that UV light can enter at least a part of the oxidation flow path 34 from outside.

One end of the oxidation flow path 34 is connected to a sample water supply flow path 28 provided as a through hole, and the other end of the oxidation flow path 34 is connected to the sample flow path 31 through a through hole 26 provided in the substrate 21. The sample flow path 31 is connected to a through hole 30 provided in the substrate 21 to discharge sample water and to a discharge port 29 provided as a through hole 30 in the substrate 24.

The oxidation flow path 34 can be provided by forming a fine groove (width: 0.01 to several mm, depth: 0.01 to 1 mm, length: several tens of millimeters to several hundred millimeters) on the upper surface of the substrate 21. For example, a fine groove having a width of 1 mm, a depth of 0.2 mm, and a length of 200 mm is used. The flow path can be formed by a wet etching technique, a dry etching technique, or a fine processing technique using sandblasting.
As the substrate 24 made of a material transparent to UV light so that UV light can enter the oxidation flow path 34, for example, a silica glass substrate is used. As the substrate 21, a silicon substrate having an oxide film is used. These substrates 24 and 21 can be bonded together by, for example, hydrofluoric acid bonding.

The supply flow path 28 and discharge port 29 for supplying and discharging sample water into and from the device are previously formed in the substrate 24, and the through hole 26 for introducing sample water into the device and the through hole 30 are also previously formed in the substrate 21. These supply port, discharge port, and through holes can be formed by, for example, sandblasting. One end of the oxidation flow path 34 is located at a position corresponding to the sample supply flow path 28, and the other end of the oxidation flow path 34 is connected to the through hole 26. The through hole 30 is formed at a position corresponding to the sample discharge port 29.

Finally, the substrate 21 and the substrate 22 are bonded together so that the carbon dioxide extraction section 6 is sandwiched between the organic substance oxidative decomposition section and the conductivity measuring section, and an area around the gas permeable portion 7 is sealed with the thin film 40 such as PDMS. In this way, a device for measuring total organic carbon, in which a carbon dioxide extraction section and a conductivity measuring section are integrated into one unit, is obtained. The obtained device has a reduced size and can measure TOC at a low flow rate.

The components of the device according to this embodiment, such as substrates, are made of silicon, silica glass, PDMS, and a porous fluorine resin, and therefore a problem such as elution rarely arises. Further, it is not necessary to use a pipe made of a material different from those of the components to connect adjacent sections to each other and thereby to reduce the influence of contamination resulting from elution of materials from pipes. In addition, it is also possible to reduce a dead volume between adjacent sections and thereby to efficiently carry out measurement with high sensitivity.

Hereinbelow, operations for measuring the concentration of organic substance using the measuring device according to this embodiment will be described with reference to Fig. 4.
As sample water, for example, an aqueous potassium biphthalate solution is used. The sample water is supplied into the oxidation flow path 34 through the supply flow path 28 at a flow rate of about 0.1 mL/min. The sample water is irradiated with UV light for 0.1 to 5 minutes, preferably 3 minutes so that organic substance contained in the sample water is oxidized and then dissolved as carbon dioxide in the sample water.

Ion exchange water is supplied from the ion exchange resin column 10 through the supply flow path 37 at a flow rate of 0.1 to 10 mL/min. In this embodiment, ion exchange water is supplied at a flow rate of 2 mL/min. However, since the flow rate of measurement water at the time of measuring conductivity is very low, a surplus of the ion exchange water supplied through the supply flow path 37 flows through the bypass flow path 15a and is then discharged through the branched discharge port 39 to control the flow rate of measurement water. By providing the bypass flow path 15a and the discharge port 39, it is possible to optimize the flow rate of an ion exchange water circulating system including the ion exchange resin column 10 (to 1.9 mL/min) as well as to control the flow rate of measurement water supplied into the measurement flow path 16a (e.g., to 0.1 mL/min).

The sample water containing dissolved carbon dioxide derived from organic substance is sent into the sample flow path 31 through the through hole 26, and then carbon dioxide dissolved in the sample water passes through the gas permeable portion 7 to ion exchange water flowing through the measurement flow path 16a.
The sample water, from which carbon dioxide has been removed by the gas permeable portion 7, flows through the through hole 30 and is then discharged through the discharge port 29. The measurement water containing carbon dioxide is sent to the measuring cell through the through hole 25. Then, the measurement water flows through the flow path 33 so that the conductivity of the ion exchange water is measured by the measuring electrode 38.

At this time, the measurement water discharged from the detection section 11 is sucked into the syringe pump 12 in a state where the open/close valve 13 is closed, and therefore the flow rate of the measurement water flowing through the carbon dioxide extraction section 6 and the detection section 11 is controlled by the syringe pump 12 to be almost constant
After the completion of measurement, the open/close valve 13 is opened, and then the measurement water in the syringe pump 12 is returned to the pure water tank 8 to prepare for the next measurement
Sample water not containing organic substance and carbon dioxide at all is also subjected to measurement to measure a background. The background is subtracted from the measurement result of sample water, and then the concentration of carbon dioxide is determined based on conductivity, and the thus determined carbon dioxide concentration is converted into TOC.

The embodiment described above is merely an example of the present invention, and the materials of the substrates and the sealing material are not particularly limited as long as similar functions can be obtained. Further, the structure of the measuring device is not limited to one constituted from the four substrates 21, 22, 23, and 24. For example, the measuring device may be constituted from only the substrates 21, 22, and 23, or only the substrates 21 and 22. Even in a case where the measuring device is constituted from, for example, only the substrates 21, 22, and 23, measurement can be carried out in the same manner as in the case of the embodiment described above by introducing a sample containing carbon dioxide derived from organic substance contained in sample water through the through hole 26. Further, the device for measuring total organic carbon according to the present invention may be one other than such an integrated measuring device formed by stacking.

Although the measuring electrode of the measuring device according to this embodiment has a comb-teeth shape, another measuring electrode such as a parallel plate type measuring electrode formed by providing electrodes on the substrates 22 and 23 may be used.
Further, as shown in Fig. 3, the measuring device according to the embodiment shown in Fig. 4 may have a flow path switching valve 18 instead of the open/close valve 13 at the branch point

### INDUSTRIAL APPLICABILITY

The present invention can be applied to a device for measuring total organic carbon for measuring the amount of total organic carbon (TOC) contained in sample water containing few impurities by measuring conductivity and a µTAS.

### BRIEF DESCRIPTION OF THE DRAWINGS

Fig. 1 is a schematic view of a device for measuring total organic carbon according to the present invention.
Fig. 2 is a graph showing variations in measured conductivity.
Fig. 3 is a schematic view showing a branch point at which a measurement flow path branches into a syringe pump and a discharge flow path.
Fig. 4 is a sectional view of a device for measuring total organic carbon.
Fig. 5 is a schematic view of a device for measuring total organic carbon as a comparative example.

### DESCRIPTION OF THE REFERENCE NUMERALS

- 1: IC removal section
- 2: Hydrophobic porous membrane
- 3: Vacuum pump
- 4: Organic substance oxidative decomposition section
- 5: UV lamp
- 6: Carbon dioxide extraction section
- 7: Gas permeable membrane
- 8: Pure water tank
- 9: Electromagnetic pump
- 10: Ion exchange resin column
- 11: Detection section
- 12: Syringe pump
- 13: Open/close valve
- 14: Liquid delivery flow path
- 15: Bypass flow path
- 16: Measurement flow path
- 17: Discharge flow path
- 18: Flow path switching valve

## Claims

1. A device for measuring total organic carbon comprising:
an organic substance oxidative decomposition section for converting organic carbon contained in sample water into carbon dioxide;
a carbon dioxide extraction section for extracting carbon dioxide generated in the organic substance oxidative decomposition section into measurement water through a gas permeable portion; and
a detection section for measuring the conductivity of the measurement water discharged from the carbon dioxide extraction section to determine the amount of the extracted carbon dioxide,
wherein a syringe pump is provided downstream of the detection section in a measurement flow path through which measurement water flows to pass through the carbon dioxide extraction section and the detection section, and
wherein the flow rate of measurement water during measurement of conductivity in the detection section is controlled by suction into the syringe pump.

2. The device for measuring total organic carbon according to claim 1,
wherein the measurement flow path through which measurement water flows to pass through the detection section branches into the syringe pump and a discharge flow path, and
wherein on the discharge flow path side located downstream of a branch point between the syringe pump and the discharge flow path in the measurement flow path, an open/close valve controlled to be closed during suction into the syringe pump and to be opened during discharge from the syringe pump is provided.

3. The device for measuring total organic carbon according to claim 1 or 2,
wherein the measurement flow path through which measurement water flows to pass through the detection section branches into the syringe pump and a discharge flow path, and
wherein at a branch point between the syringe pump and the discharge flow path, a switching valve controlled so that the syringe pump is connected to the detection section during suction into the syringe pump and so that the syringe pump is connected to the discharge flow path during discharge from the syringe pump is provided.

4. The device for measuring total organic carbon according to any one of claims 1 to 3,
wherein an ion exchange resin column is provided on the upstream side in a liquid delivery flow path for supplying measurement water into the carbon dioxide extraction section,
wherein the liquid delivery flow path branches into the measurement flow path and a bypass flow path at a position located downstream of the ion exchange resin column, and
wherein the flow rate of measurement water to be supplied into the measurement flow path through the liquid delivery flow path is controlled by the split function of the bypass flow path.

5. The device for measuring total organic carbon according to claim 4, wherein the discharge side of the measurement flow path and the discharge side of the bypass flow path are connected to the measurement water supply side of the ion exchange resin.

6. The device for measuring total organic carbon according to any one of claims 1 to 6,
wherein the carbon dioxide extraction section is formed by stacking a gas permeable portion through which carbon dioxide contained in sample water passes, a first substrate fixed to the gas permeable portion so as to be opposed thereto and having a flow path through which the sample water flows, and a second substrate fixed to the gas permeable portion so as to be opposed thereto and having a flow path through which the measurement water flows.

7. The device for measuring total organic carbon according to claim 6,
wherein a third substrate is fixed to the second substrate so as to be opposed thereto to form a flow path between the second substrate and the third substrate and an electrode is provided as the detection section in the flow path, and
wherein a fourth substrate is fixed to the first substrate so as to be opposed thereto to form a flow path between the first substrate and the fourth substrate and the organic substance oxidative decomposition section is constituted from the flow path and an irradiation portion for irradiating the flow path with UV light

8. The device for measuring total organic carbon according to any one of claims 1 to 7, wherein the carbon dioxide extraction section uses, as the gas permeable portion, any one selected from the group consisting of a membrane filter, a porous fluorine membrane, a hollow fiber membrane, and a membrane having a plurality of fine flow paths.
